# DEMANDE DE BREVET EUROPEEN

(11) **EP 2 140 943 A1**
(43) Date de publication de la demande: **06.01.2010**
(21) Numéro de dépôt: 09163251.3
(22) Date de dépôt: 19.06.2009
(51) Int. Cl.: B05B 17/06, A61K 8/00

(54) **Atomiseur piézoélectrique comprenant une composition liquide parfumante et procédé de parfumage**

(30) Priorité: 02.07.2008 FR 0854482
(71) Demandeur: L'Oréal, 75008 Paris (FR)
(72) Inventeur: Bara, Isabelle, 94210, LA VARENNE ST HILAIRE (FR); Albelda, Virginie, 75013, PARIS (FR); Charbonnier-Civier, Carole, 91170, VIRY CHATILLON (FR)

(57) **Abrégé**

L'invention se rapporte à un ensemble cosmétique comprenant :
(A) une composition cosmétique ou dermatologique contenue à l'intérieur d'un réservoir, ladite composition étant liquide et comprenant, dans un milieu physiologiquement acceptable,
(i) de 1 à 90% en poids d'un concentré de parfum ;
(ii) de 1 à 98,5% en poids d'au moins un solvant non volatil dudit concentré ayant une densité inférieur à 1 ;
(iii) au moins de 0,5 à 50% en poids d'au moins un alcool volatil dudit concentré ; ; ladite composition ayant une viscosité inférieure ou égale à 8mPa.s; les quantités étant définies par rapport au poids total de la composition ;

(B) un dispositif de pulvérisation comprenant un récipient contenant ladite composition cosmétique liquide, équipé d'un mécanisme de pulvérisation piézoélectrique permettant de pulvériser la composition cosmétique sous forme de gouttelettes.

L'invention se rapporte également à un procédé de parfumage des matières kératiniques humaines et notamment de la peau, des lèvres, des cheveux, du cuir chevelu, les ongles, comprenant au moins une étape de pulvérisation d'une composition parfumante au moyen d'un dispositif tel que défini précédemment.

L'invention se rapporte également à un procédé de parfumage de l'air ambiant comprenant au moins une étape de pulvérisation d'une composition parfumante au moyen d'un dispositif de pulvérisation tel que défini précédemment.

## Description

La présente invention a pour objet un dispositif de pulvérisation du type piézoélectrique comprenant une composition liquide parfumante comprenant elle-même au moins un concentré de parfum, au moins un solvant non-volatil et un solvant volatil.

Un concentré de parfum est un mélange de matières odorantes. Il correspond à un mélange d'éléments naturels et synthétiques.

Le concentré de parfum est composé d'une multitude d'odeurs qui sont perçues simultanément et /ou successivement et que les parfumeurs ont l'habitude de classer selon trois catégories :
Note de tête
Note de coeur
Note de fond.
Cette classification est réalisée selon la volatilité de chaque élément.

Les notes de têtes sont les plus volatiles, il s'agit généralement de notes fraîches, légères, vertes et fruitées. Elles correspondent au premier sentiment olfactif du produit et sont responsable de l'acte d'achat. Leurs volatilités est tellement importante que leurs présences durent jusqu'à 30 minutes environ.

Les notes de coeur correspondent aux notes intermédiaires en termes de volatilité, elles apparaissent après les notes de têtes, elles constituent le coeur du parfum, elles vont être senties intensément au cours de la journée et constituent l'identité majeure du parfum, même si c'est la synergie des trois parties de la pyramide qui crée l'identité globale.

Les notes de fond, sont des notes lourdes qui appuient le parfum. Elles permettent de stabiliser les notes de têtes et de coeur. Elles créent une base solide au parfum. Elles peuvent comprendre les odeurs ambrées, musquées, boisées, balsamiques. Ces notes sont peu volatiles et peuvent persister au-delà de 5 heures. Elles correspondent généralement aux notes qui persistent des jours durant sur le textile de nos vêtements.

Une fois le concentré de parfum élaboré selon une pyramide olfactive précise, il peut être en général mis en solution alcoolique, hydro-alcoolique pour donner le Parfum.

La qualité de l'odeur est un critère hédonique important, mais d'autres critères plus techniques, tels que la diffusion, l'intensité, la persistance et la rémanence sont à considérer.

L'être humain a de tout temps cherché à se parfumer et à parfumer les objets qui l'entourent ou les lieux dans lesquels il se trouve, et ceci, aussi bien pour masquer des odeurs fortes et/ou désagréables que pour donner une bonne odeur.

Le Parfum s'applique sur différents supports.
1) En cosmétique : par exemple en hygiène corporelle (gel douche, shampoings...), produits parfumants (Eaux fraîches, Eaux de toilette, Eaux de parfum, Extraits, Produits de soins et produits parfumés, visage et corps, Maquillage) ;
2) en parfumerie fonctionnelle (Détergents et Parfums d'ambiance), pour donner dans tous les cas des produits ayant un atout parfum.

Différents moyens permettent la déposition du parfum sur la peau ou sur d'autres supports comme l'air ambiant pour les Air Fresheners.

La parfumerie fait généralement appel aux flacons pompes pour les eaux de toilettes, à des gaz propulseurs pour les déodorants ou la parfumerie détergente.

Concernant les cosmétiques c'est la base elle-même qui lors de son application sur la peau dépose le parfum, exemple des crèmes, laits ou concrètes.
La parfumerie fonctionnelle et la parfumerie d'ambiance concerne les bougies, l'encens, les céramiques parfumées, les bouquets parfumés, et les diffuseurs de parfum.

Les diffuseurs électriques appelés « plugg-in » se branchent directement sur secteur, et la technologie tend à proposer de plus en plus des diffuseurs de type piezoélectrique qui fonctionnent à l'aide de batterie pour un avantage « nomade », mais qui surtout offrent une fine pulvérisation largement plus esthétique et olfactivement supérieure en terme de qualité de restitution des notes de parfum. Ce dernier système pouvant s'utiliser pour de nombreux produits parfums ou cosmétiques si la viscosité est adaptée.

De nombreux diffuseurs de type piézoélectrique permettant de disperser une composition parfumante ont été proposés. La méthode consiste à faire vibrer une membrane de céramique, de consistance poreuse, par exemple munie d'orifices traversant, qui va faire diffuser sous forme de microgouttelettes la composition parfumante qui est amenée jusqu'à ce point par une mèche. Il est déjà connu des documents WO03/066115, WO00/53337, US5,518,179, WO02/089861 WO06/066671, WO07/054920 d'utiliser des plaques vibrantes pour disperser un produit sous forme d'un nuage de fines gouttelettes. Les dispositifs décrits dans ces documents comportent un réservoir de produit liquide et un système d'éjection comportant une paroi munie d'un ou plusieurs orifices pour le passage du produit. En variante, il est également connu du document WO02068128 des dispositifs comportant un élément piézoélectrique qui est prévu au niveau d'une paroi opposée à la paroi munie d'orifices de distribution. Un élément piézoélectrique est prévu pour, lorsqu'il se déforme sous l'effet d'un champ électrique, faire vibrer la paroi de manière à faire sortir le produit sous forme de fines gouttelettes au travers des orifices. Dans ces dispositifs, le produit est amené au niveau des orifices de la paroi par une mèche redit qui trempe dans le réservoir de produit.

On connaît dans la demande WO00/47335 des diffuseurs de parfum de type piézoélectrique comprenant une composition parfumante liquide de viscosité inférieure à 10 cps et ayant une tension de surface de 20 à 35 dynes/cm afin d'optimiser le rendement de la diffusion et de minimiser la consommation de la batterie électrique alimentant le dispositif.

Les formulations de parfum présentes dans ces types de dispositif comprennent généralement un concentré de parfum comprenant des composés organiques volatils (VOC), au moins un solvant dudit parfum ; la viscosité de la composition liquide étant adaptée pour permettre la diffusion du dit parfum.

Les diffuseurs de parfum du type piézoélectrique peuvent conduire à un certain nombre d'inconvénients qu'il convient d'éviter ou du moins d'en diminuer les effets néfastes comme
- le phénomène dit de « Fall Out » qui se traduit par des retombées de gouttelettes sur la surface de l'appareil ou sur son support ;
- l'encrassement ou l'obstruction de l'appareil ;
- une hauteur et un volume de nuage de la composition vaporisée généré par le dispositif (« pshitt ») désagréables visuellement pour le consommateur ;
- une évaporation incontrôlée de la solution parfumante de son réservoir ou cartouche entraînant une longévité du produit non-satisfaisante pour le consommateur ;
- le ou les solvants utilisés peuvent produire une odeur qui affecte le parfum recherché.

Aussi, il subsiste donc le besoin de rechercher de nouvelles formulations conditionnées dans des diffuseurs de parfum du type piézoélectrique dans lesquels les qualités de diffusion et la qualité olfactive sont améliorées, ainsi que la longévité du produit sans les inconvénients énoncés précédemment.

La demanderesse au cours de ses nombreuses recherches a découvert de manière surprenante que cet objectif pouvait être atteint en utilisant dans un dispositif de pulvérisation du type piézoélectrique une composition liquide comprenant, dans un milieu physiologiquement acceptable,
(i) de 1 % à 90% en poids (de préférence 5 à 30%) d'un concentré de parfum ;
(ii) de 1 à 98,5%% en poids d'au moins un solvant non volatil dudit concentré de parfum ayant une densité inférieure à 1 ;
(iii) au moins de 0,5 % à 50 % en poids d'au moins un solvant volatil ; ; la dite composition ayant une viscosité inférieure ou égale à 8mPa.s.

Cette découverte est à la base de l'invention.

L'invention a donc pour objet un ensemble comprenant :
(A) une composition parfumante contenue à l'intérieur d'un réservoir, ladite composition étant liquide et comprenant, dans un milieu physiologiquement acceptable,
   (i) de 1 % à 90%en poids (de préférence 5 à 30%) d'un concentré de parfum ;
   (ii) de 1 à 98,5%% en poids d'au moins un solvant non volatil dudit parfum ayant une densité inférieure à 1;
   (iii) au moins de 0,5% à 50% en poids d'au moins un solvant volatil dudit parfum; ladite composition ayant une viscosité inférieure à 8mPa.s ; les quantités étant définies par rapport au poids total de la composition.
(B) un dispositif de pulvérisation comprenant un récipient contenant ladite composition cosmétique liquide d'un mécanisme de pulvérisation piézo-électrique permettant de pulvériser la composition parfumante sous forme de gouttelettes.

L'invention a encore pour objet un procédé de parfumage des matières kératiniques humaines et notamment de la peau, des lèvres, des cheveux, du cuir chevelu, les ongles, comprenant au moins une étape de pulvérisation d'une composition parfumante au moyen d'un dispositif tel que défini précédemment.

L'invention a encore pour objet un procédé de parfumage de l'air ambiant comprenant au moins une étape de pulvérisation d'une composition parfumante au moyen d'un dispositif tel que défini précédemment.

Par composition parfumante, on entend toute composition, mélange de concentré pur, de solvants et d'additifs nécessaire à la conservation de la composition.

Par « concentré de parfum», on entend toute substance odorante sous sa forme simple ou sous forme de mélange, les deux formes possibles comprenant les solvants nécessaires à la transformation de la matière naturelle et/ou à l'obtention du mélange. Le concentré de parfum appliqué sur un support donne une composition parfumante. Ce support peut être cosmétique, dermatologique, alcoolique, hydro-alcoolique, ou mélange de solvants.

Par « solvant volatil », on entend tout solvant du concentré de parfum ayant une pression de vapeur supérieure ou égale à 0,01 kPa à 293,15°K ou bien supérieure à 0,1 mm de Hg.

Par « Composé Organique Volatil », on entend tout composé organique ayant une pression de vapeur supérieure ou égale à 0,01 kPa à 293,15°K ou bien supérieure à 0,1 mm de Hg.

La viscosité de la composition est mesurée à 20°C au moyen d'un appareil Rhéostress 600 -« HAAKE » avec mobile ayant 60 mm de diamètre, un angle de 2° et un revêtement sablé à un taux de cisaillement de 200 s⁻¹.

On entend par milieu physiologiquement acceptable dans la composition de l'invention, un milieu non toxique et susceptible d'être appliqué sur les matières kératiniques humaines comprenant la peau, le visage, les lèvres, les ongles, les cheveux, le cuir chevelu ou bien susceptible d'être diffusé dans l'atmosphère, dans l'air ambiant.

### Dispositif de pulvérisation piézoélectrique

Par système de pulvérisation piézoélectrique, on entend, au sens de la présente demande un système qui nébulise un liquide sous l'action d'une énergie ultrasonique de fréquence et de puissance appropriées, énergie produite par un matériau piézoélectrique (transducteur) excité par un signal électrique à haute fréquence.

Selon un mode de réalisation préférentiel de l'invention, le dispositif de pulvérisation piézoélectrique peut comprendre :
- une membrane perforée, les perforations de la membrane faisant communiquer l'intérieur du récipient avec l'environnement externe,
- un actionneur pour faire vibrer la membrane,
- un moyen pour amener la composition cosmétique liquide contenue dans le
récipient au contact d'une surface interne de la membrane, la composition cosmétique, sous l'effet des vibrations de la membrane s'écoulant à travers les perforations jusqu'à une surface externe de la membrane d'où elle émerge sous forme de gouttelettes.

Les perforations dans la membrane ont de préférence, une conicité inversée, c'est-à-dire une surface en coupe transversale plus grande sur la surface externe de la membrane, faisant face à l'environnement externe, que sur la surface interne, faisant face à l'intérieur du récipient.

Le dispositif de pulvérisation peut comprendre en outre un moyen de décalage de pression, tel que décrit dans la demande WO95/15822, fournissant une pression réduite au liquide en contact avec la surface interne de la membrane. La pression réduite peut varier d'une pression nulle jusqu'à la pression à laquelle l'air est aspiré à travers les perforations de la membrane en contact avec la composition.

De préférence, les perforations, sur la surface externe de la membrane, ne se touchent pas.

De préférence encore, l'actionneur est un actionneur piézo-électrique, par exemple conçu pour faire vibrer la membrane dans une gamme de fréquence pouvant aller par exemple 20 KHz à 7 MHz. L'énergie nécessaire au fonctionnement de l'actionneur piézo-électrique peut être obtenue grâce à un générateur électrique, par exemple une pile électrique, une batterie ou une cellule photovoltaïque pouvant être éventuellement couplé à un circuit électronique.

Dans le dispositif de pulvérisation défini ci-dessus, le moyen pour amener la composition cosmétique liquide à la surface de la membrane peut comprendre un mécanisme d'alimentation par capillarité, ou alternativement, un mécanisme d'alimentation à générateur de bulles ou encore une pompe de type péristaltique, à membrane, à piston, à engrenages. De tels mécanismes sont décrits par exemple dans la demande internationale WO95/15822.

Selon un mode de réalisation particulier de l'invention, toutes les perforations ont une conicité inversée, ou à l'inverse, la membrane comporte, de plus, des perforations de conicité normale.

Par perforation de conicité normale, on entend au sens de la présente invention des perforations dont la surface en coupe transversale est plus petite sur la surface externe de la membrane, faisant face à l'environnement externe, que sur la surface interne, faisant face à l'intérieur du récipient.

Lorsque des perforations de conicité normale sont présentes, celles-ci sont de préférence disposées autour et à l'extérieur des perforations de conicité inversée.

Le moyen pour amener la composition cosmétique liquide à la surface de la membrane peut être conçu pour amener ladite composition sur la surface interne de ladite membrane, ou à l'inverse, être conçu pour amener ladite composition sur la surface externe de ladite membrane. De telles variantes du dispositif de pulvérisation sont décrites, par exemple, dans la demande internationale WO95/15822.

A titre d'exemple, la membrane peut être formée d'un disque circulaire d'un diamètre de 8 mm, de nickel electroformé, d'une épaisseur de 70µm possédant une pluralité de perforations. Les perforations peuvent présenter une surface en coupe transversale en forme de disque circulaire dont le diamètre varie de 4 à 150µm sur la surface externe de la membrane, faisant face à l'environnement externe, et sur la surface interne, faisant face à l'intérieur du récipient, une surface en coupe transversale en forme de disque circulaire dont le diamètre varie de 2 à 50µm, et par exemple de 10 à 20µm.

Lors de l'utilisation du dispositif, la composition cosmétique émerge sous forme de gouttelettes dont le diamètre moyen est compris de préférence entre 20 et 100µm, et de manière encore plus préférée entre 30 et 60 µm.

Des technologies correspondant à ce dispositif ont notamment été décrites dans les demandes WO 93/10910, US 5, 487, 378, FR-A-2665572, US 4,533,082 ; US5518179, US6113001, WO06/066671.

Pour la diffusion d'une composition parfumée, il peut s'avérer souhaitable de disposer d'un ensemble permettant un remplacement aisé d'une cartouche contenant un produit à diffuser par une autre cartouche contenant un produit différent, tout en ayant des cartouches réalisées à un coût compatible avec une diffusion à grande échelle.

Selon un mode particulier de l'invention, on peut utiliser un ensemble de pulvérisation tel que divulgué par WO06/125677. Il s'agit d'un dispositif de pulvérisation comportant un anneau réalisé au moins partiellement en matériau piézoélectrique et une membrane perforée mise en vibration par l'anneau, dispositif dans lequel la membrane perforée est maintenue dans l'anneau par serrage ; le dispositif piézoélectrique comportant un récipient pour alimenter la membrane perforée en produit à pulvériser.

Selon un autre mode particulier de l'invention, on peut utiliser un ensemble de pulvérisation tel que celui divulgué par US 4 702 418. Cet ensemble comporte un appareil pourvu d'un organe excitateur et une cartouche reçue dans l'appareil, comportant une chambre de pression délimitée d'un côté par une paroi déformable venant au contact de l'organe excitateur et de l'autre côté par une grille perforée par laquelle des gouttelettes de produit sont éjectées, dans l'axe de la région de la paroi déformable venant au contact de l'organe excitateur. L'organe excitateur peut être solidaire d'un logement recevant la cartouche.

### Concentré de parfum

Dans le concentré de parfum utilisé conformément à l'invention, on peut utiliser dans la composition de l'invention, les parfums d'origine naturelle ou synthétique et leurs mélanges. Comme parfums d'origine naturelle, on peut citer par exemple les extraits de fleurs (lis, lavande, rose, jasmin, ilang-ilang), de tiges et de feuilles (patchouli, géranium, petit-grain), de fruits (coriandre, anis, cumin, genièvre), d'écorces de fruits (bergamote, citron, orange), de racines (angélique, céleri, cardamome, iris, acore), de bois (bois de pin, santal, gaïac, cèdre, rose), d'herbes et de graminées (estragon, lemon grass, sauge, thym), d'aiguilles et de branches (épicéa, sapin, pin, pin nain), de résines et de baumes (galbanum, élémi, benjoin, myrrhe, oliban, opopanax).

Comme substance parfumante d'origine synthétique, on peut citer par exemple les composés du type ester, éther, aldéhyde, cétone, alcool aromatique et hydrocarbure.

Comme esters, on peut citer en particulier l'acétate de benzyle, le benzoate de benzyle, l'isobutyrate de phénoxyéthyle, l'acétate de p-tert-butylcyclohexyle, l'acétate de citronellyle, le formiate de citronellyle, l'acétate de géranyle, l'acétate de linalyle, l'acétate de diméthyl-benzylcarbinyle, l'acétate de phényléthyle, le benzoate de linalyle, le formiate de benzyle, le glycinate d'éthylméthylphényle, le propionate d'alkylcyclohexyle, le propionate de styralyle et le salicylate de benzyle.

Comme éthers, on peut citer le benzyléthyléther.

Comme aldéhydes, on peut citer par exemple les alcanes linéaires comportant de 8 à 18 atomes de carbone, le citral, le citronellal, le citronellyloxyacétaldéhyde, le cyclamènaldéhyde, l'hydroxycitronellal, le lilial et le bourgeonal.

Comme cétones, on peut citer par exemple les ionones comme l'alpha-isométhylionone, et la méthylcédrylcétone.

Parmi les alcools aromatiques et notamment terpéniques, on peut citer l'anéthol, le citronellol, l'eugénol, l'isoeugénol, le géraniol, le linalol, le phényléthylalcool et le terpinéol.

Comme hydrocarbures, on peut citer notamment les terpènes tels que par exemple le menthol, le nérol et le limonène.

Par ailleurs, on peut aussi utiliser des huiles essentielles, composants d'arômes, comme par exemple les essences de sauge, de camomille, de girofle, de mélisse, de menthe, de feuilles de cannelier, de fleurs de tilleul, de genièvre, de vétiver, d'oliban, de galbanum, de labdanum et de lavandin.

On utilise de préférence comme substance parfumante, seule ou en mélange, l'essence de bergamote, le dihydromyrcénol, le lilial, le lyral, le citronellol, l'alcool phényléthylique, l'alpha-hexylcinnamaldéhyde, le géraniol, la benzylacétone, le cyclamènaldéhyde, le linalol, l'ambroxan, l'indol, l'hédione, la sandelice, les essences de citron, de mandarine et d'orange, le glycolate d'allylamine, le cyclovertal, l'essence de lavandin, l'essence de sauge, le bétadamascone, l'essence de géranium, le salicylate de cyclohexyle, l'acide phénylacétique, l'acétate de géranyle, l'acétate de benzyle, l'oxyde de rose.

Selon un mode préféré de réalisation de l'invention, on utilise un mélange de différentes substances parfumantes qui engendrent par leur synergie en commun une note plaisante pour l'utilisateur. Parmi les notes olfactives connues, on peut citer par exemple les parfums hespéridés, les aromatiques, les parfums floraux, les musqués, les parfums fruités, les épicés, les parfums orientaux, les parfums marins, les notes aquatiques, les parfums chyprés, les parfums boisés, les fougères et leurs mélanges

La viscosité de la composition parfumante est inférieure ou égale à 8 mPa.s et variera en fonction de son application (peau ou air ambiant). Elle variera de préférence de 1 à 8 mPa.s. Celle-ci est mesurée à 20°C au moyen d'un appareil Rhéostress 600 -« HAAKE » avec mobile ayant 60 mm de diamètre, un angle de 2° et un revêtement sablé à un taux de cisaillement de 200 s⁻¹

### Solvants non volatils du parfum

Les solvants non volatils conformes à la présente invention sont de préférence choisis parmi les esters, les éthers, les polyols, les alcools gras.

Leur viscosité variera de préférence de 1 à 120 m Pa.s. Celle-ci est mesurée à 20°C au moyen d'un appareil Rhéostress 600 -« HAAKE » avec mobile ayant 60 mm de diamètre, un angle de 2° et un revêtement sablé à un taux de cisaillement de 200 s^{-1.}

Leur densité est inférieure à 1. Celle-ci peut être mesurée à 20°C au moyen d'un densimètre du type METTLER TOLEDO DE 45.

A titre d'exemple de solvants non volatils, on peut citer le néopentanoate d'octyl-2-dodecyle, le néopentanoate d'iso-décyle, le dicaprilyl éther.

On utilisera plus particulièrement le 3-méthoxy-3-methyl-1-butanol et le myristate d'isopropyle.

### Solvant volatil

Les solvants volatils conformes à l'invention sont choisis de préférence parmi les alcools volatils.

Par « alcool volatil », tout composé comprenant au moins une fonction alcool ayant une pression de vapeur à 20 °C supérieure à 17,5 mm de mercure.

Les alcools volatils conformes à la présente invention sont choisis de préférence parmi les monoalcools inférieurs en C₁-C₅ comme par exemple le méthanol, l'éthanol, propanol, l'isopropanol, le n-butanol, l'isobutanol, le t-butanol et plus particulièrement l'éthanol. Leur viscosité variera de préférence de 0,5 à 3 m Pa.s. Celle-ci est mesurée à 20°C au moyen d'un appareil Rhéostress 600 - « HAAKE » avec mobile ayant 60 mm de diamètre, un angle de 2° et un revêtement sablé à un taux de cisaillement de 200 s⁻¹.

La concentration en solvant volatil est de 0,5% à 50% en poids par rapport au poids total de la composition. En dessous de 0,5% en poids la diffusion du parfum n'est pas réalisable. Plus préférentiellement, elle variera de 0,5 à 25% et encore plus préférentiellement de 0,5 à 16% en poids.

### Additifs

La composition de l'invention peut comprendre, en outre, tout additif usuellement utilisé dans le domaine des parfums choisi notamment parmi les antioxydants, les filtres UV, les stabilisants, des matières colorantes, les conservateurs (par exemple phénoxyéthanol et parabènes), les bactéricides ou leurs mélanges.

Parmi les antioxydants, on peut citer par exemple le BHA (tert-butyl-4-hydroxyanisole), le BHT (2,6-di-tert-butyl-p-crésol), les tocophérols comme la vitamine E et ses dérivés tels que l'acétate de tocophéryle.

La composition selon l'invention peut notamment comprendre au moins une matière colorante notamment les colorants liposolubles et les colorants hydrosolubles.

Les colorants solubles sont par exemple : le caramel, Yellow 5 , Acid Blue 9/ Blue 1, Green 5, Green 3 / Fast Green FCF 3, Orange 4, Red 4 / Food Red 1, Yellow 6, Acid Red 33 / Food Red 12, Red 40, le carmine de cochenille (CI 15850, CI 75470), Ext. Violet 2, Red 6-7, Ferric Ferrocyanide, Ultramarines, Acide Yellow 3 / Yellow 10, Acid Blue 3, Yellow 10.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.

La composition selon l'invention peut ainsi constituer une composition de parfumage sous forme d'eau fraîche, d'eau de toilette, d'eau de parfum, extraits à fort pourcentage en concentré de parfum, de lotion après-rasage, d'eau de soin et plus particulièrement de parfum d'ambiance.

L'invention a encore pour objet un procédé cosmétique de parfumage des matières kératiniques des êtres humains et notamment de la peau, des lèvres et des phanères, comprenant l'application sur les matières kératiniques de la composition telle que définie ci-dessus.

La composition selon l'invention peut être fabriquée par les procédés connus, généralement utilisés dans le domaine des compositions parfumées.

Les compositions parfumantes selon l'invention se présenter sous toutes les formes galéniques adaptées aux diffuseurs du type piézoélectrique notamment sous forme de solution alcoolique ou hydro-alcoolique. Ces compositions sont préparées selon les méthodes usuelles.

L'invention va maintenant être décrite en référence aux exemples suivants donnés à titre illustratif et non limitatif. Dans ces exemples, sauf indication contraire, les quantités sont exprimées en pourcentages pondéraux. On a réalisé les formulations parfumées suivantes ; les quantités sont indiquées en pourcentages en poids :

### Exemple1 : Influence de la viscosité de la composition sur la diffusion :

On réalise les ensembles formulation + atomiseur piézoélectrique 1 à 8 suivants

| **Ingrédients** | **EX 1** | **EX 2** | **EX 3** | **EX 4** | **EX 5** | **EX 6** | **EX 7** | **EX 8** |
|---|---|---|---|---|---|---|---|---|
| Parfum Sésame soufflé | 50 | 50 | 20 | 10 | - | - | - | - |
| Parfum Zan Violette | - | - | - | - | 15 | 15 | 10 | 7 |
| Ethanol | 12 | 12 | 15 | 16 | 17 | 17 | 17 | 17 |
| 3-methoxy-3-methyl-1-butanol (7 mPa.s) | 38 | - | - | - | 68 | - | - | - |
| Myristate d'isopropyle (5,3 mPa.s) | - | 38 | 65 | 74 | | 68 | 73 | 76 |
| **Viscosité de la composition (mPa.S)** | **8.4** | **6.8** | **4.6** | **4.3** | **5.8** | **4.7** | **4.3** | **4.2** |
| **Diffusion** | **-** | **+** | **+++** | **++++** | **+** | **+++** | **++++** | **++++** |

On incorpore chacune de ces formulations parfumantes dans un dispositif de pulvérisation piézoélectrique tel que décrit dans la demande par WO06/125677 comportant un e cartouche pour alimenter la membrane perforée en produit à pulvériser. Ladite cartouche est remplie à l'aide de pipette Rainin, avec 1ml de la composition à tester. La diffusion est réalisée durant 30 minutes continues, avec contrôle toutes les 10 minutes. On définit la qualité de la diffusion obtenue de la manière suivante :

| | |
|---|---|
| **-** | Pas de diffusion, ou encrassement de l'appareil très rapide (en moins de 10 secondes) |
| **+** | Diffusion moyenne |
| **++** | Diffusion correcte |
| **+++** | Bonne diffusion |
| **++++** | Très bonne diffusion : volume important, hauteur importante, pas d'encrassement de l'appareil. |

### Exemple N°2 : Effet de la densité du solvant non volatil :

On réalise les ensembles formulation + atomiseur piézoélectrique 15 à 20 suivants :

Mesure de la densité à 20°C à l'aide de l'appareil METTLER TOLEDO DE 45. La mesure de la densité des solutions à diffuser par le système met en évidence des familles de solutions regroupées selon le solvant mis en solution et quelques soit leurs viscosités. On incorpore chacune de ces solutions parfumantes dans un dispositif de pulvérisation piézoélectrique. Identique à celui de l'exemple 1. La cartouche est remplie à l'aide d'une pipette Rainin, avec 1 ml de la solution à tester.

La fréquence de l'appareil est fixée à
Temps de diffusion = 300 millisecondes
Temps d'arrêt= 1 seconde

| **Ingrédients** | **EX 15** | **EX 16** | **EX 17** | **EX 18** | **EX 19** | **EX 20** |
|---|---|---|---|---|---|---|
| Parfum Tiaré Taïti | 28 | 28 | 28 | 28 | 28 | - |
| Parfum Pétale de Vétiver | - | - | - | - | - | 50 |
| Ethanol non dénaturé | 3 | 23 | 30 | 40 | 3 | 15,5 |
| Citrate de triéthyle (36mPa.s) | - | 49 | 42 | | - | - |
| Dipropylène glycol (119 mPa.s) | - | - | - | 32 | - | |
| Néopentanoate d'octyldodécyle (17mPa.s) | - | - | - | - | - | |
| Myristate d'isopropyle (5,3mPa.s) | 69 | - | - | - | 69 | |
| 3-methoxy-3-methyl-1-butanol (7 mPa.s) | - | - | - | - | - | 34,5 |
| Viscosité (mPa.S) | 5,7 | 5,7 | 4,5 | 5 | 5,7 | 5,4 |
| Densité du solvant non volatil | 0,85 | 1,13 | 1,13 | 1,02 | 0.85 | 0.92 |
| **Diffusion** | **+++** | **-** | **-** | **-** | **+++** | **+++** |

Ces résultats montrent que lorsque la densité du solvant non volatil est supérieure à 1, la diffusion ne s'effectue pas.

### Exemple n°3 : Influence du solvant volatil :

Ces essais sont réalisés afin de montrer l'influence de l'ajout du solvant volatil sur la diffusion de la solution au sein de l'appareil.

On réalise les ensembles formulation + atomiseur piézoélectrique 9 à 14 suivants

| **Ingrédients** | **EX 9** | **EX 10** | **EX 11** | **EX 12** | **EX 13** | **EX 14** |
|---|---|---|---|---|---|---|
| Parfum Jingle Bell | 10 | 10 | 10 | 10 | 10 | 10 |
| Ethanol non dénaturé | - | 0,4 | 0,5 | - | 0,4 | 0,5 |
| Myristate d'isopropyle (5,3mPa.s) | qsp 100 | qsp 100 | qsp 100 | - | - | - |
| 3-methoxy-3-methyl-1-butanol (7 mPa.s) | | | | qsp 100 | qsp 100 | qsp 100 |
| **Viscosité (mPa.S)** | **5,53** | **5,49** | **5,5** | **7** | **5,39** | **5,52** |
| **Diffusion** | **-** | **-** | **+** | **-** | **-** | **+** |

On incorpore chacune de ces solutions parfumantes dans un dispositif de pulvérisation piézoélectrique identique à celui de l'exemple 1. La cartouche est remplie à l'aide d'une pipette Rainin, avec 1 ml de la solution à tester.

La fréquence de l'appareil est fixée à
Temps de diffusion = 300 millisecondes
Temps d'arrêt= 1 seconde.

Ces résultats montrent que lorsque la teneur en solvant volatil est en dessous de 0,5% en poids de solvant volatil, la diffusion ne s'effectue pas.

### Exemple n°4 : Longévité des formulations

On réalise les ensembles formulation + atomiseur piézoélectrique 21 à 25 suivants

| **Ingrédients** | **EX 21** | **EX 22** | **EX 23** | **EX 24** | **EX 25** |
|---|---|---|---|---|---|
| Parfum Tiaré Taïti | 10 | 10 | 10 | 10 | 10 |
| Ethanol non dénaturé | 10 | 16 | 50 | 75 | 90 |
| Myristate d'isopropyle | qsp 100 | qsp 100 | qsp 100 | qsp 100 | qsp 100 |

On incorpore chacune de ces solutions parfumantes dans un dispositif de pulvérisation piézoélectrique identique à celui de l'exemple 1. La cartouche est remplie à l'aide d'une pipette Rainin, avec 1 ml de la composition à tester.
On mesure le pourcentage d'évaporation des solutions introduites dans les cartouches placées à l'étuve 37°C.
Les pesées sont réalisées à T=

| | | | | | | | | |
|---|---|---|---|---|---|---|---|---|
| **Temps (heures)** | 1 | 3.66 | 7 | 24 | 48 | 72 | 144 | 168 |

### On obtient les résultats suivants :

### Résultats :

| **temps (heures)** | **% évap 10% éthanol** | **% évap 16% éthanol** | **% évap 50% éthanol** | **% évap 75% éthanol** | **% évap 90% éthanol** |
|---|---|---|---|---|---|
| 1 | 1,97 | 2,2 | 3,78 | 5,6 | 4,11 |
| 3,66 | 5,66 | 6,52 | 9,48 | 11,82 | 13,79 |
| 7 | 8,43 | 10,99 | 15,97 | 19,19 | 25,73 |
| 24 | 10,45 | 16,24 | 44,45 | 53,55 | 80,16 |
| 48 | 10,71 | 16,74 | 50,45 | 74,91 | 90,32 |
| 72 | 10,88 | 16,98 | 50,89 | 75,57 | 90,66 |
| 144 | 11,2 | 17,49 | 51,59 | 76,29 | 91,25 |
| 168 | 11,25 | 17,61 | 51,72 | 76,41 | 91,39 |

### Conclusion :

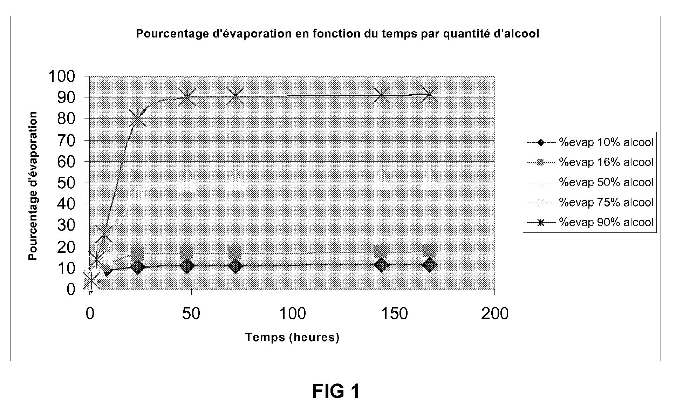

Le graphique de la figure 1 montre que les courbes atteignent un plateau dès 48 heures de test à l'étuve (37°C). Ce test à l'étuve est réalisé pour accélérer le processus d'évaporation général à température ambiante. Le maximum d'évaporation pour toutes les solutions est atteint. Si l'on se fixe un maximum d'évaporation de 50% pour éviter toute déception du consommateur et proposer un produit de qualité, on constate qu'il ne faut pas aller au-delà de 50% d'alcool et préférentiellement 16%.

## Revendications

1. Ensemble comprenant :
(A) une composition contenue à l'intérieur d'un réservoir, ladite composition étant liquide et comprenant, dans un milieu physiologiquement acceptable au moins :
(i) de 1 à 90% en poids d'un concentré de parfum ;
(ii) de 1 à 98,5% en poids d'au moins un solvant non volatil dudit concentré ayant une densité inférieure à 1;
dudit parfum ;
(iii) au moins de 0,5% à 50% en poids d'au moins un solvant volatil dudit concentré; ladite composition ayant une viscosité inférieure ou égale 8 mPa.s ; les quantités étant définies par rapport au poids total de la composition.
(B) un dispositif de pulvérisation comprenant un récipient contenant ladite composition cosmétique liquide, équipé d'un mécanisme de pulvérisation piézo-électrique permettant de pulvériser la composition cosmétique sous forme de gouttelettes.

2. Ensemble selon la revendication 1, **caractérisé en ce que** le récipient équipé d'un mécanisme de pulvérisation comprend :
- une membrane perforée, les perforations de la membrane faisant communiquer l'intérieur du récipient avec l'environnement externe,
- un transducteur piézoélectrique,
- un moyen pour amener la composition cosmétique liquide contenue dans le récipient au contact d'une surface interne de la membrane,
la composition cosmétique, sous l'effet des vibrations s'écoulant à travers les perforations jusqu'à une surface externe de la membrane, d'où elle émerge sous forme de gouttelettes.

3. Ensemble selon la revendication 2, **caractérisé en ce que** le transducteur piézoélectrique fait vibrer ladite membrane.

4. Ensemble selon l'une quelconque des revendications 1 à 3, comportant un dispositif de pulvérisation piézoélectrique comportant un anneau réalisé au moins partiellement en matériau piézoélectrique et une membrane perforée mise en vibration par l'anneau, dispositif dans lequel la membrane perforée est maintenue dans l'anneau par serrage ; le dispositif piézoélectrique comportant un récipient pour alimenter la membrane perforée en produit à pulvériser.

5. Ensemble selon l'une quelconque des revendications 1 à 3, comportant un dispositif de pulvérisation piézoélectrique pourvu d'un organe excitateur et une cartouche reçue dans l'appareil, comportant une chambre de pression délimitée d'un côté par une paroi déformable venant au contact de l'organe excitateur et de l'autre côté par une grille perforée par laquelle des gouttelettes de produit sont éjectées, dans l'axe de la région de la paroi déformable venant au contact de l'organe excitateur ; l'organe excitateur pouvant être solidaire d'un logement recevant la cartouche.

6. Ensemble selon l'une quelconque des revendications 1 à 3, où le solvant volatil est choisi parmi les mono-alcools inférieurs en C₁-C₅ en particulier l'éthanol.

7. Ensemble selon l'une quelconque des revendications 1 à 6, où la viscosité du solvant volatil varie de 0,5 à 3 mPa.s.

8. Ensemble selon l'une quelconque des revendications 1 à 7, où la concentration en solvant volatil varie de 0,5 à 25% et encore plus préférentiellement de 0,5 à 16% en poids.

9. Ensemble selon l'une quelconque des revendications 1 à 7, où la viscosité de la composition parfumante liquide est de 0,5 à 8 mPa.s.

10. Ensemble selon la revendication 8 où la concentration en solvant volatil varie de 0,5 à 25% et encore plus préférentiellement de 0,5 à 15% en poids.

11. Ensemble selon l'une quelconque des revendications 1 à 10, où le solvant non volatil du parfum est choisi parmi les esters, les éthers, les polyols, les alcools gras en particulier choisi parmi le 3-méthoxy-3-methyl-1-butanol et le myristate d'isopropyle.

12. Ensemble selon l'une quelconque des revendications 1 à 11, où la viscosité du solvant non volatil du parfum est de 5 à 120 mPa.s.

13. Ensemble selon l'une quelconque des revendications 1 à 12, où la composition liquide contenue dans le dispositif de pulvérisation se présente sous forme d'eau fraîche, d'eau de toilette, d'eau de parfum, d'extrait de parfum, de lotion après-rasage, d'eau de soin ou de parfum d'ambiance.

14. Procédé de parfumage des matières kératiniques humaines et notamment de la peau, des lèvres, des cheveux, du cuir chevelu, les ongles, comprenant au moins une étape de pulvérisation d'une composition parfumante au moyen d'un dispositif tel que défini dans l'une quelconque des revendications 1 à 13.

15. Procédé de parfumage de l'air ambiant comprenant au moins une étape de pulvérisation d'une composition parfumante au moyen d'un dispositif tel que défini dans l'une quelconque des revendications 1 à 13.
